# EUROPEAN PATENT APPLICATION

(11) **EP 3 320 944 A1**
(43) Date of publication of application: **16.05.2018**
(21) Application number: 17200645.4
(22) Date of filing: 08.11.2017
(51) Int. Cl.: A61M 25/10, A61B 18/14, A61B 5/042, A61B 5/06

(54) **MULTI-ELECTRODE CATHETER FOR PREVENTING PHYSIOLOGICAL FLUID FLOW RESTRICTION**

(30) Priority: 09.11.2016 US 201662419930 P; 25.10.2017 US 201715793572
(71) Applicant: Biosense Webster (Israel) Ltd., 2066717 Yokneam (IL)
(72) Inventor: RUBINSTEIN, Vladimir, 2066717 Yokneam (IL); AUERBACH, Shmuel, 2066717 Yokneam (IL); ULTCHIN, Yigal, 2066717 Yokneam (IL); BAR-TAL, Meir, 2066717 Yokneam (IL); HOLANDER, Ludwig Eliezer, Haifa 3365159 (IL)
(74) Representative: Carpmaels & Ransford LLP

(57) **Abstract**

A medical apparatus, used to acquire electrical activity of patient anatomy, is provided which includes an elongated body and a tip portion coupled to the elongated body. The tip portion includes one or more inflatable sections. Each inflatable section has a plurality of electrodes disposed on one of: (i) an outer surface of the one or more inflatable sections; and (ii) an inner surface and the outer surface of the one or more inflatable sections. The one or more inflatable sections, when inflated, cause a portion of the plurality of electrodes to contact a surface of an organ and provide a pathway for physiological fluid to flow through the tip portion. In one embodiment, the tip portion is a tulip balloon tip portion. In another embodiment, the tip portion is an inflatable tip portion having one or more concentrically wound inflatable sections.

## Description

### CROSS REFERENCE TO RELATED APPLICATION

This application claims the priority benefit of U.S. Provisional Application No. 62/419,930, filed on November 9, 2016, which is incorporated herein by reference as if fully set forth.

### SUMMARY

The present application provides a medical apparatus used to acquire electrical activity of patient anatomy. The apparatus includes an elongated body and a tip portion coupled to the elongated body. The tip portion includes one or more inflatable sections, each having a plurality of electrodes disposed on one of (i) an outer surface of the one or more inflatable sections; and (ii) an inner surface and the outer surface of the one or more inflatable sections. The one or more inflatable sections, when inflated, cause a portion of the plurality of electrodes to contact a surface of an organ and provide a pathway for physiological fluid to flow through the tip portion.

The present application provides a medical apparatus, used to acquire electrical activity of patient anatomy, which includes an elongated body and a tulip balloon tip portion coupled to the elongated body. The tulip balloon tip portion includes a plurality of inflatable sections configured to provide a pathway for physiological fluid to flow through the tip portion. The inflatable sections have a plurality of electrodes disposed on at least one of: (i) an outer surface of the plurality of inflatable sections; and (ii) an inner surface and the outer surface of the plurality of inflatable sections. The inflatable sections include a first inflatable section and a second inflatable section. The second inflatable section is configured to overlap the first inflatable section in a first state.

The present application provides a medical apparatus, used to acquire electrical activity of patient anatomy, which includes an elongated body and an inflatable tip portion coupled to the elongated body. The inflatable tip portion includes one or more concentrically wound inflatable sections, each having a plurality of electrodes disposed on at least one of (i) an outer surface of the one or more concentrically wound inflatable sections; and (ii) an inner surface and the outer surface of the one or more concentrically wound inflatable sections. When inflated, the one or more concentrically wound inflatable sections are configured to form a gap to provide a pathway for physiological fluid to flow and the plurality of electrodes are caused to contact an inner surface of an organ.

### BRIEF DESCRIPTION OF THE DRAWINGS

For a better understanding of the present invention, reference is made to the detailed description of the invention, by way of example, which is to be read in conjunction with the following drawings, wherein like elements are given like reference numerals.
FIG. 1 is an illustration of an example medical system for navigating a tool in a three dimensional (3-D) space according to embodiments disclosed herein;
FIG. 2 is an illustration of components of an example medical system for use with embodiments described herein;
FIG. 3 is an illustration of an example tulip balloon portion in a first state according to an embodiment;
FIG. 4 is an illustration of the example tulip balloon portion shown in FIG. 3 in a second state according to an embodiment;
FIG. 5 is an illustration of an example inflatable tip portion, having a concentrically wound inflatable section, in a first state according to an embodiment;
FIG. 6 is an illustration of the example inflatable tip portion shown in FIG. 5 in a second state according to an embodiment;
FIG. 7 is a top view of an example electrode array according to an embodiment;
FIG. 8 is a side view of the example electrode array shown in FIG. 7;
FIG. 9 is an illustration of an electrode array having electrodes grouped to form a larger electrode for ablation according to an embodiment; and
FIG. 10 is an illustration of an electrode array having equidistant electrodes grouped to form a larger electrode for ablation according to an embodiment.

### DETAILED DESCRIPTION OF THE INVENTION

Catheter ablation techniques (e.g., radio frequency (RF) ablation) include ablating portions of dysfunctional tissue, such as tissue of a heart, stomach, lung, ear, nose, throat and other organs. Some catheter ablation techniques are used for treating cardiac arrhythmia, such as atrial fibrillation (AFib), in which the heart tissue is ablated to terminate electrical pathways and block faulty electrical impulses that can cause heart rhythm disorders. For example, a catheter may be inserted through an incision in the skin and guided to the heart where the catheter is used to create ablation lesions on the heart tissue.

Before ablation of heart tissue is performed, catheters may also be used to detect electrical activity of the heart for generating maps (e.g., high resolution maps) of the heart. For example, intra-cardiac (IC) electrocardiogram (ECG) signals of the heart are acquired (i.e., recorded for a period of time, such as 20 to 30 seconds) via a plurality of catheter electrodes placed at different areas of the heart. The acquired signals are monitored and used, along with location information indicating locations of the electrodes in a three dimensional (3D) space, to create the dynamic maps of the heart. Based on a visual assessment of the maps, a region of interest (ROI) of the heart may be determined, which may include an area of the heart causing an irregular heart rhythm to be targeted for ablation.

Conventional catheters used to generate the heart maps and ablate organ tissue include basket catheters and balloon catheters. Basket catheters typically place a number of electrodes in contact with the surface of the heart such that the electrode coverage of the cardiac chamber is sufficient for providing accurate mapping of the electrical activity of the heart. The electrodes of these basket catheters may not, however, conform well to the wall of the cardiac chamber. The nonconformity is substantial in the left atrium which has a complex shape. Accordingly, some basket electrodes suffer from poor contact (e.g., partial or no contact) with the cardiac chamber such that electrical signals are not acquired via these electrodes.

Balloon catheters are placed within a cardiac chamber and include portions which inflate to enlarge an opening or passage of the heart. The electrodes of these balloon catheters typically provide better contact with the cardiac chamber than basket catheters. The dimensions of balloon catheters are typically much smaller than the dimensions of the mapped cardiac cavity, however, to prevent physiological fluid (e.g., blood) flow restriction (i.e., blocking blood flow). Accordingly, balloon catheters provide less electrode coverage of the cardiac chamber than basket catheters, which may result in a less accurate representation of the electrical activity of the heart.

The present application discloses catheters having inflatable components configured to prevent physiological fluid flow restriction while maintaining electrode coverage of the cardiac chamber sufficient for providing accurate mapping of the electrical activity of the heart. Embodiments include catheters used for mapping of the heart as well as for both mapping of the heart and ablating cardiac tissue.

Embodiments described herein obtain accurate electrical activity measurements to map the cardiac chamber in a single heartbeat and over a period of time. Embodiments enable high density mapping in accordance with the cavity mapping capability, such that the catheter does not need to be moved to different locations within the heart cavity to acquire the electrical activity data to map the heart cavity.

Systems, apparatuses and methods which facilitate ablation contour control are also disclosed herein. For example, embodiments include electrode arrays, having a plurality of individual electrodes, configured on catheter surfaces, to provide high resolution mapping as well as being combinable into one or more electrode groups, each acting as a larger electrode, facilitating the creation of ablation outlines for ablation.

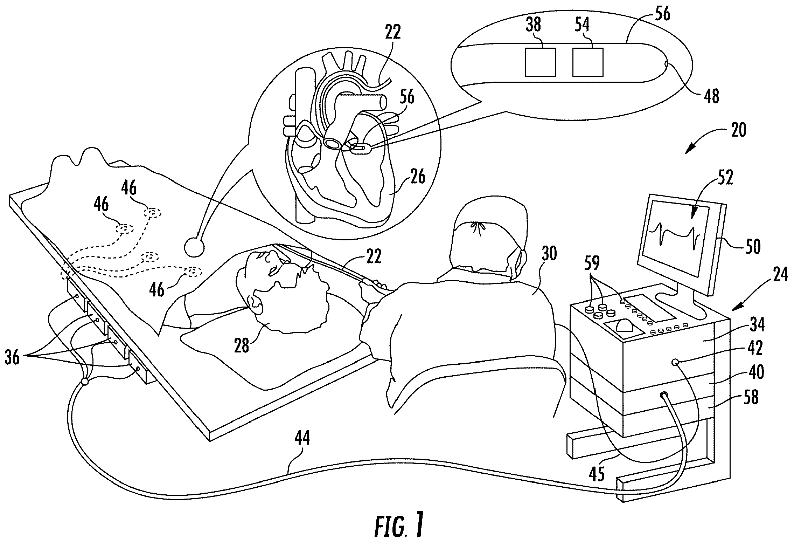

Referring now to FIG. 1, an illustration of an example medical system 20 is shown that may be used to generate and display information 52 (e.g., anatomical models of a portion of a patient and signal information). Tools, such as tool 22 can be any tool used for diagnostic or therapeutic treatment, such as for example, a catheter having a plurality of electrodes for mapping electrical potentials in a heart 26 of a patient 28. Alternatively, tools may be used, mutatis mutandis, for other therapeutic and/or diagnostic purposes of different portions of anatomy, such as in the heart, stomach, or other body organs.

An operator 30 may insert the tool 22 into a portion of patient anatomy, such as the vascular system of the patient 28 so that a tip 56 of the tool 22 enters a chamber of the heart 26. The control console 24 may use magnetic position sensing to determine 3-D position coordinates of the tool (e.g., coordinates of the tip 56) inside the heart 26. To determine the position coordinates, a driver circuit 34 in the control console 24 may drive, via connector, 44, field generators 36 to generate magnetic fields within the anatomy of the patient 28.

The field generators 36 include one or more emitter coils (not shown in FIG. 1), placed at known positions external to the patient 28, which are configured to generate magnetic fields in a predefined working volume that contains a portion of interest of the patient anatomy. Each of the emitting coils may be driven by a different frequency to emit a constant magnetic field. For example, in the example medical system 20 shown in FIG. 1, one or more emitter coils can be placed below the torso of the patient 28 and each configured to generate magnetic fields in a predefined working volume that contains the heart 26 of the patient.

As shown in FIG. 1, a magnetic field location sensor 38 may be disposed at the tip 56 of tool 22. The magnetic field location sensor 38 generates electrical signals, based on the amplitude and phase of the magnetic fields, indicating the 3-D position coordinates of the tool (e.g., position coordinates of the tip 56). The electrical signals may be communicated to the control console 24 to determine the position coordinates of the tool. The electrical signals may be communicated to the control console 24 via wire 45.

Alternatively, or in addition to wired communication, the electrical signals may be wirelessly communicated to the control console 24, for example, via a wireless communication interface (not shown) at the tool 22 that may communicate with input/output (I/O) interface 42 in the control console 24. For example, U.S. Pat. No. 6,266,551, whose disclosure is incorporated herein by reference, describes, inter alia, a wireless catheter, which is not physically connected to signal processing and/or computing apparatus and is incorporated herein by reference. Rather, a transmitter/receiver is attached to the proximal end of the catheter. The transmitter/receiver communicates with a signal processing and/or computer apparatus using wireless communication methods, such as IR, RF, Bluetooth, or acoustic transmissions. The wireless digital interface and the I/O interface 42 may operate in accordance with any suitable wireless communication standard that is known in the art, such as for example, IR, RF, Bluetooth, one of the IEEE 802.11 family of standards (e.g., Wi-Fi), or the HiperLAN standard.

Although FIG. 1 shows a single magnetic field location sensor 38 disposed at the tip 56 of tool 22, tools may include one or more magnetic field location sensors each disposed at any tool portion. The tools (e.g., catheters) described herein may also be used without a 3-D mapping system. The magnetic field location sensor 38 may include one or more miniature coils (not shown). For example, a magnetic field location sensor may include multiple miniature coils oriented along different axes. Alternatively, the magnetic field location sensor may comprise either another type of magnetic sensor or position transducers of other types, such as impedance-based or ultrasonic location sensors.

The signal processor 40 is configured to process the signals to determine the position coordinates of the tool 22, including both location and orientation coordinates. The method of position sensing described hereinabove is implemented in the CARTO mapping system produced by Biosense Webster Inc., of Diamond Bar, Calif., and is described in detail in the patents and the patent applications cited herein.

The tool 22 may also include a force sensor 54 contained within the tip 56. The force sensor 54 may measure a force applied by the tool 22 (e.g., the tip 56 of the tool) to the endocardial tissue of the heart 26 and generate a signal that is sent to the control console 24. The force sensor 54 may include a magnetic field transmitter and a receiver connected by a spring in the tip 56, and may generate an indication of the force based on measuring a deflection of the spring. Further details of this sort of probe and force sensor are described in U.S. Patent Application Publications 2009/0093806 and 2009/0138007, whose disclosures are incorporated herein by reference. Alternatively, the tip 56 may include another type of force sensor that may use, for example, fiber optics or impedance measurements.

The tool 22 may also include an electrode 48 coupled to the tip 56 and configured to function as an impedance-based position transducer. Additionally or alternatively, the electrode 48 may be configured to measure a certain physiological property, for example the local surface electrical potential (e.g., of cardiac tissue) at one or more locations. The electrode 48 may be configured to apply RF energy to ablate endocardial tissue in the heart 26.

Although the example medical system 20 may be configured to measure the position of the tool 22 using magnetic-based sensors, other position tracking techniques may be used (e.g., impedance-based sensors). Magnetic position tracking techniques are described, for example, in U.S. Pat. Nos. 5,391,199, 5,443,489, 6,788,967, 6,690,963, 5,558,091, 6,172,499 6,177,792, the disclosures of which are incorporated herein by reference. Impedance-based position tracking techniques are described, for example, in U.S. Pat. Nos. 5,983,126, 6,456,828 and 5,944,022, the disclosures of which are incorporated herein by reference.

The I/O interface 42 may enable the control console 24 to interact with the tool 22, the body surface electrodes 46 and any other sensors (not shown). Based on the electrical impulses received from the body surface electrodes 46 and the electrical signals received from the tool 22 via the I/O interface 42 and other components of medical system 20, the signal processor 40 may determine the location of the tool in a 3-D space and generate the display information 52, which may be shown on a display 50.

The signal processor 40 may be included in a general-purpose computer, with a suitable front end and interface circuits for receiving signals from the tool 22 and controlling the other components of the control console 24. The signal processor 40 may be programmed, using software, to perform the functions that are described herein. The software may be downloaded to the control console 24 in electronic form, over a network, for example, or it may be provided on non-transitory tangible media, such as optical, magnetic or electronic memory media. Alternatively, some or all of the functions of the signal processor 40 may be performed by dedicated or programmable digital hardware components.

In the example shown at FIG. 1, the control console 24 is connected, via cable 44, to body surface electrodes 46, each of which are attached to patient 28 using patches (e.g., indicated in FIG. 1 as circles around the electrodes 46) that adhere to the skin of the patient. Body surface electrodes 46 may include one or more wireless sensor nodes integrated on a flexible substrate. The one or more wireless sensor nodes may include a wireless transmit/receive unit (WTRU) enabling local digital signal processing, a radio link, and a miniaturized rechargeable battery. In addition or alternative to the patches, body surface electrodes 46 may also be positioned on the patient using articles worn by patient 28 which include the body surface electrodes 46 and may also include one or more position sensors (not shown) indicating the location of the worn article. For example, body surface electrodes 46 can be embedded in a vest that is configured to be worn by the patient 28. During operation, the body surface electrodes 46 assist in providing a location of the tool (e.g., catheter) in 3-D space by detecting electrical impulses generated by the polarization and depolarization of cardiac tissue and transmitting information to the control console 24, via the cable 44. The body surface electrodes 46 can be equipped with magnetic location tracking and can help identify and track the respiration cycle of the patient 28. In addition to or alternative to wired communication, the body surface electrodes 46 may communicate with the control console 24 and one another via a wireless interface (not shown).

During the diagnostic treatment, the signal processor 40 may present the display information 52 and may store data representing the information 52 in a memory 58. The memory 58 may include any suitable volatile and/or non-volatile memory, such as random access memory or a hard disk drive. The operator 30 may be able to manipulate the display information 52 using one or more input devices 59. Alternatively, the medical system 20 may include a second operator that manipulates the control console 24 while the operator 30 manipulates the tool 22. It should be noted that the configuration shown in FIG. 1 is exemplary. Any suitable configuration of the medical system 20 may be used and implemented.

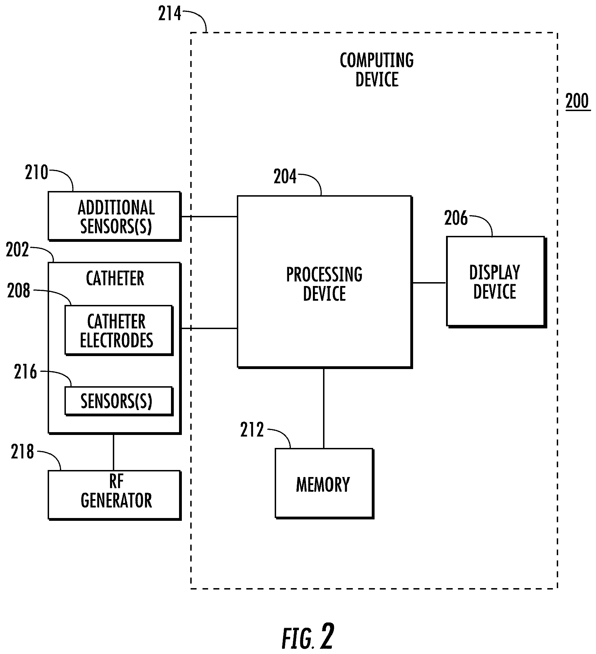

FIG. 2 is a block diagram illustrating example components of a medical system 200 in which features described herein can be implemented. As shown in FIG. 2, the system 200 includes catheter 202, processing device 204, display device 206 and memory 212. As shown in FIG. 2, the processing device 204, display device 206 and memory 212 are a part of computing device 214. In some embodiments, the display device 206 may be separate from computing device 214. Computing device 214 may also include an I/O interface, such as I/O interface 42 shown in FIG. 1.

As shown in FIG. 2, catheter 202 includes a plurality of catheter electrodes 208 for detecting the electrical activity of the heart over time. Catheter 202 may also include one or more sensors 216, which include, for example, sensors (e.g., a magnetic field location sensor) for providing location signals to indicate the location of the catheter 202 in a 3-D space as well as sensors (e.g., position sensors, pressure or force sensors, temperature sensors, impedance sensors) for providing ablation parameter signals during the ablation of the heart tissue. The example system 200 also includes one or more additional sensors 210, separate from the catheter 202, used to provide location signals indicating the location of the catheter 202 in a 3D space.

The system 202 shown in example system 200 also includes an RF generator 218, which supplies high-frequency electrical energy, via catheter 202, for ablating tissue at locations engaged by the catheter 202. Accordingly, catheter 202 may be used to acquire electrical activity for generating mapping of the heart as well ablating cardiac tissue. As described above, however, embodiments may include catheters used to acquire the electrical activity for generating mapping of the heart while not used to ablate cardiac tissue.

Processing device 204 may include one or more processors each configured to process the ECG signals, record ECG signals over time, filter ECG signals, fractionate ECG signals into signal components (e.g., slopes, waves, complexes) and generate and combine ECG signal information for displaying the plurality of electrical signals on display device 206. Processing device 204 may also generate and interpolate mapping information for displaying maps of the heart on display device 206. Processing device 204 may include one or more processors (e.g., signal processor 40) configured to process the location information acquired from sensors (e.g., additional sensor(s) 210 and catheter sensor(s) 216) to determine location and orientation coordinates.

Processing device 204 is also configured to drive display device 206 to display dynamic maps (i.e., spatio-temporal maps) of the heart and the electrical activity of the heart using the mapping information and the ECG data. Display device 206 may include one or more displays each configured to display maps of the heart representing spatio-temporal manifestations of the electrical activity of the heart over time and display the ECG signals acquired from the heart over time.

The catheter electrodes 208, catheter sensor(s) 216 and additional sensor(s) 210 may be in wired or wireless communication with processing device 204. Display device 206 may also be in wired or wireless communication with processing device 204.

The processing device 204 may be guided by operator 30 to connect one or more electrodes 208 of catheter 202 to the RF generator 218 for creating a lesion with a guided shape on the inner surface of heart 26 (e.g. a curve).

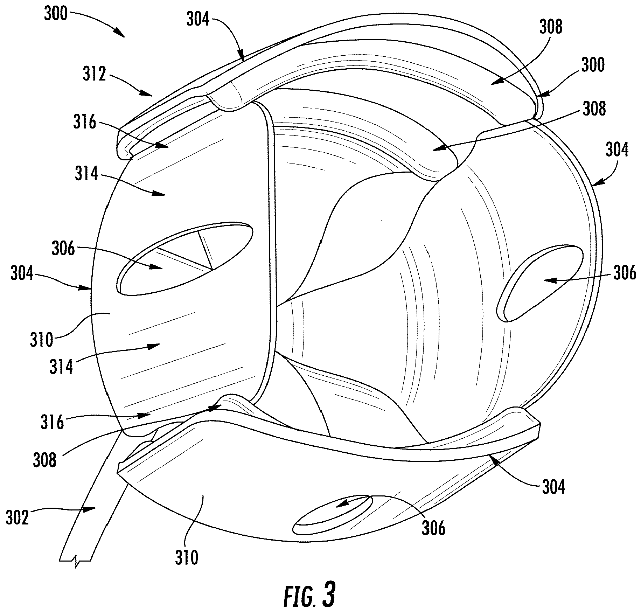

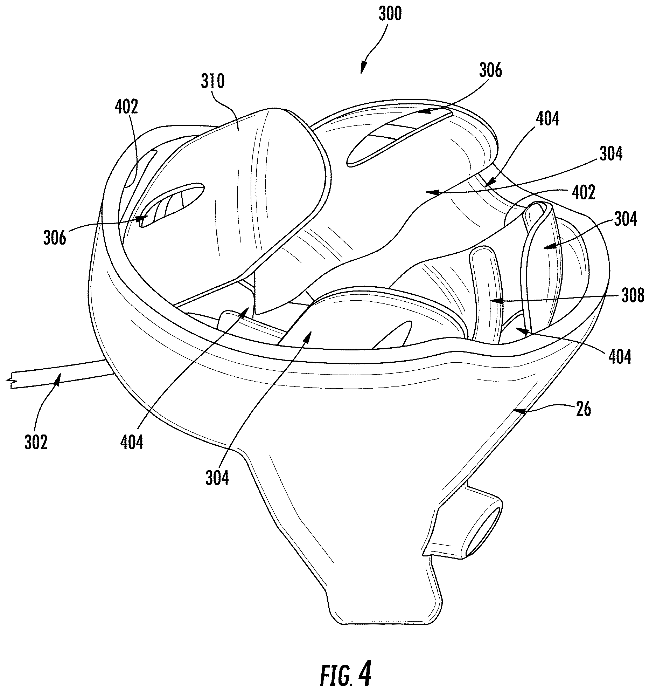

FIGS. 3 and 4 illustrate different states of an example tulip balloon portion 300 of a tool, such as catheter 202. The tulip balloon portion 300 is located, for example, at a tip of catheter body portion 302 in the form of an elongated shaft. As shown in FIG. 3, the tulip balloon portion 300 includes four inflatable sections 304, each shaped similar to the shape of a tulip petal. The number of inflatable sections shown in FIGS. 3 and 4 is merely exemplary. Tulip balloon portions may include a different number of inflatable sections (e.g., less than or more than four sections). In addition, the shape of the inflatable sections 304 shown in FIGS. 3 and 4 is exemplary. Tulip inflatable sections may be shaped differently from than the inflatable sections 304 shown in FIGS. 3 and 4. Inflatable sections 304 can be configured using polyurethane.

As shown in FIG. 3, the four inflatable sections 304 include a pair of opposing first inflatable sections 304 (i.e., left and right sections in FIG. 3) and a pair of opposing second inflatable sections 304 (i.e., top and bottom sections in FIG. 3). Each of the inflatable sections 304 include a hole 306, located at each inflatable section 304 such that a center of an inflatable section 306 is within a corresponding hole 306. The shapes, locations and number of holes 306 shown in FIGS. 3 and 4 are, however, merely exemplary. For example, numerous holes may be disposed at different locations at each inflatable section 304.

In some embodiments, one or more inflatable sections may include protrusions for providing a pathway for the physiological fluid to flow through the holes 306. For example, as shown in FIG. 3, each of the pair of opposing second inflatable sections 304 include protrusions, in the form of two ridges 308, protruding from an inner surface of a balloons section 304 and extending between opposing ends of an inflatable section 304. The shapes, locations and number of ridges 308 shown in FIGS. 3 and 4 are also exemplary. Inflatable section 304 may include any number of protrusions, such as ridges, configured to provide a pathway for the physiological fluid to flow through the holes 306. In some embodiments, tulip balloon portions do not include any protrusions.

Each of the inflatable sections 304 includes a plurality of electrodes (e.g., 200 electrodes) disposed on its outer surface 310, which are used, for example, to acquire electrical signals at different areas of an organ (e.g., heart 26) over time. Electrodes are not shown in FIGS. 3 and 4 to better illustrate other features of the tulip balloon portion 300 (e.g., features of the inflatable section 304). It can be appreciated, however, that electrodes may be disposed at different locations of the tulip balloon portion 300. For example, electrodes may be disposed on both an outer surface 310 of an inflatable section 304, as well as disposed on both the outer surface and an inner surface of the inflatable section 304 (e.g., an electrode disposed on an inner surface can be used as reference to an electrode on the outer surface for measuring unipolar IC ECG signals).

The inflatable sections 304 are configured such that, when inflated, the electrodes 208 disposed on the outer surfaces 310 of the inflatable sections 304, contact inner surfaces (e.g., inner surface 402 shown in FIG. 4) of the heart chamber walls to detect the electrical activity of the heart 26. Although not shown in FIGS. 3 and 4, electrodes 208 may also be disposed on the inner surface of inflatable section 304. These electrodes may be used as IC ECG reference electrodes.

Tulip balloon portion 300 may also include one or more location sensors (e.g., magnetic field location sensor 38 shown in FIG. 1) in a tip of catheter body portion 302. Location sensors are omitted from FIGS. 3 and 4 to better illustrate the features of the tulip balloon portion 300 (e.g., features of the inflatable section 304). It can be appreciated, however, that one or more location sensors may be disposed at different locations at the tulip balloon portion 300. For example, one or more of the inflated sections 304 may include a location sensor. The location sensor may be embedded within an inflated section 304 or disposed on its inner surface or its outer surface).

One or more inflatable sections of a tulip balloon portion may overlap with one or more other inflatable sections. For example, FIG. 3 illustrates each one of the pair of opposing second inflatable sections 304 of the tulip balloon portion 300 overlapping adjacent first inflatable sections 304. As shown in FIG. 3, a first end portion 312 of the second inflatable section 304 (the top inflatable section in FIG. 4) overlaps the first inflatable section 304 (the left inflatable section in FIG. 4) and a second end portion 314 of the second inflatable section 304 overlaps the first inflatable section 304 (the right inflatable section in FIG. 4).

In the first open state shown in FIG. 3, the first inflatable sections 304 are in contact with the ridges 308 of adjacent second inflatable sections 304. For example, as shown in FIG. 3, the first inflatable section 304 (the left inflatable section in FIG. 4) contacts the ridges 308 of the first end portion 312 of the second inflatable section 304 (the top inflatable section in FIG. 4) and the first inflatable section 304 (the right inflatable section in FIG. 4) contacts the ridges 308 of the second end portion 314 of the second inflatable section 304.

The inflatable sections 304 are configured with a flexible material. Accordingly, when the first inflatable sections 304 inflate and contact the ridges 308 of the second inflatable section 304, the first inflatable sections 304 flex such that middle portions (i.e., portions which include the holes 306 in FIG. 3) extend out further from the interior of the tulip balloon portion 300 than end portions of the first inflatable sections 304. If the dimensions of the heart chamber are similar (e.g., axis of the atria and the balloon are of similar length) to the dimensions of the tulip balloon portion 300 shown in the state at FIG. 3, the flexed shape of the first inflatable sections 304 cause the electrodes 208, disposed on the outer surfaces 310 of the first inflatable sections 304 at portions 314, to contact inner surfaces of the heart chamber walls. The flexed shape of the first inflatable sections 304 also cause portions 316 of the outer surfaces 310 to be spaced from the inner surfaces of the heart chamber and provide additional pathways for the physiological fluid to flow.

Dimensions for the left atrium are, for example, 50mm to 80 mm on the longitudinal axis (from septum to ridge) and 30mm to 50mm on the orthogonal axis. Dimensions for the right atriums are narrower and of similar length. In one embodiment the direction of deployment of the balloon in the left and right atria is the longest axis of the corresponding atria. The dimensions of the inflated balloon can be compliant with a full atrial size.

FIG. 4 illustrates the tulip balloon portion 300 in a second open state within a chamber of heart 26. A section of the chamber of heart 26 is not shown in FIG. 4 to illustrate the positions of the inflatable sections 304 in the second open state within the chamber of heart 26. In the second open state, the inflatable sections 304 of the tulip balloon portion 300 further open to their positions shown in FIG. 4 because the dimensions of the heart chamber are greater than the dimensions of the heart chamber described above with regard to FIG. 3. As shown in FIG. 4, when the tulip balloon portion 300 is in the second open state, the inflatable sections 304 are in contact with the inner surface 402 of the heart chamber. The inflatable sections 304 do not overlap adjacent balloons sections 304 and are spaced from each other forming gaps 404 between adjacent inflatable sections 304. Accordingly, in the second open state shown in FIG. 4, electrodes 208 (not shown in FIG. 4) disposed on the outer surfaces 310 of the inflatable sections 304 contact the inner surface 402 of the heart chamber to acquire the electrical signals of the heart 26, while the gaps 404 between the inflatable sections 304 provide additional pathways for the physiological fluid to flow.

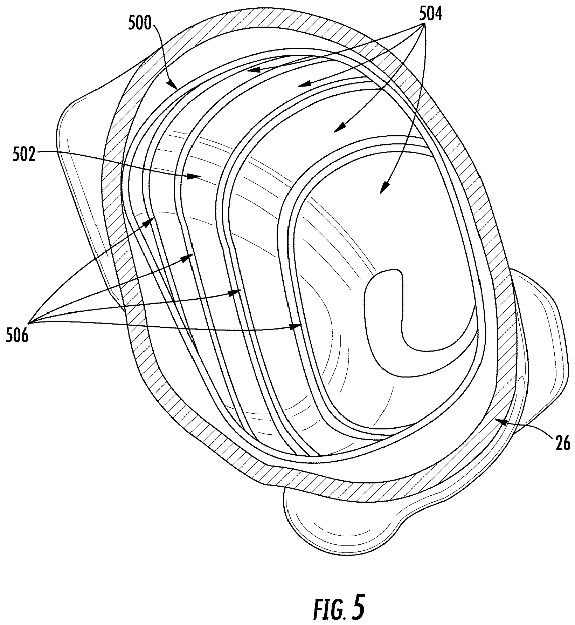

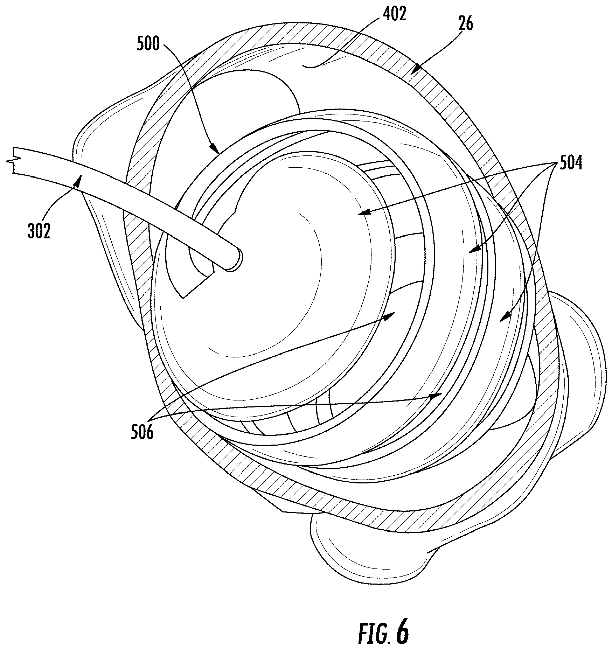

FIGS. 5 and 6 illustrate different states of an example spring-like tip portion 500 of a tool, such as catheter 202. The spring balloon portion 500 is located, for example, at a tip of catheter body portion 302. As shown in FIGS. 5 and 6, the spring balloon portion 500 includes an outer shell 502. The outer shell 502 is an inflatable strip which includes a concentrically wound inflatable section 504 (i.e., including a series of concentric bands). The example spring balloon portion 500 shown in FIGS. 5 and 6 illustrate a single concentrically wound inflatable section 504 (i.e., single helix). Embodiments may, however, include any number of concentrically wound inflatable sections (e.g., double helix, triple helix, etc.) Portions of the heart 26 are not shown in FIGS. 5 and 6 to illustrate the positions of the inflatable sections 504 within the chamber of heart 26.

The concentrically wound inflatable section 504 includes a plurality of electrodes disposed on its outer surface to acquire electrical signals at different areas of the heart 26 over time. Electrodes are not shown in FIGS. 5 and 6 to better illustrate the features of the spring balloon portion 500. It can be appreciated, however, that electrodes may be disposed at different locations at the spring balloon portion 500. For example, electrodes may be disposed on the outer surface of the concentrically wound inflatable section 504 as well as disposed on both the outer surface and the inner surface of the concentrically wound inflatable section 504. The inflatable section 504 is configured such that, when inflated, the electrodes disposed on the outer surface of the inflatable section 504, contact an inner surface 402 of the heart chamber to detect the electrical activity of the heart 26. Electrodes 208, when positioned on the outer surface of section 504 may be used for delivering RF energy to heart 26.

FIG. 5 illustrates the spring balloon portion 500 in a first state. As shown, the outer shell 502 forms a spheroid (egg-like) shape similar to the shape of the chamber of the heart 26. The outer surfaces of the concentrically wound inflatable section 504 and electrodes (not shown in FIG. 5) disposed on the outer surfaces of the concentrically wound inflatable sections 504, contact inner walls of an organ (e.g., a heart chamber). The concentrically wound inflatable section 504 separates, similar to a spring uncoiling, to form a gap 506 (e.g., continuous gap between the concentric bands) which provides pathways for the physiological fluid to flow. Accordingly, the spring balloon portion 500 provides electrode coverage sufficient for providing accurate mapping of the electrical activity of the heart (e.g., accurate electrical activity measurements are obtained to map the cardiac chamber in one heartbeat) without blocking the physiological fluid flow.

FIG. 6 illustrates the portion inflatable section 500 in a second state. In the second state, the concentrically wound inflatable sections 504 open to their positions shown in FIG. 6 because the dimensions of the heart chamber are greater than the dimensions of the heart chamber described above with regard to FIG. 5. In the second state, the outer surface of the concentrically wound inflatable section 504 contacts the inner surface 402 of the heart chamber, causing electrodes, disposed on the outer surface, to contact the inner surface 402 of the heart chamber and acquire the electrical signals. In addition, the concentric bands of the concentrically wound inflatable section 504 are further spaced from each other than in the first open state, causing the gap 506 to become larger and provide a larger pathway for the physiological fluid to flow.

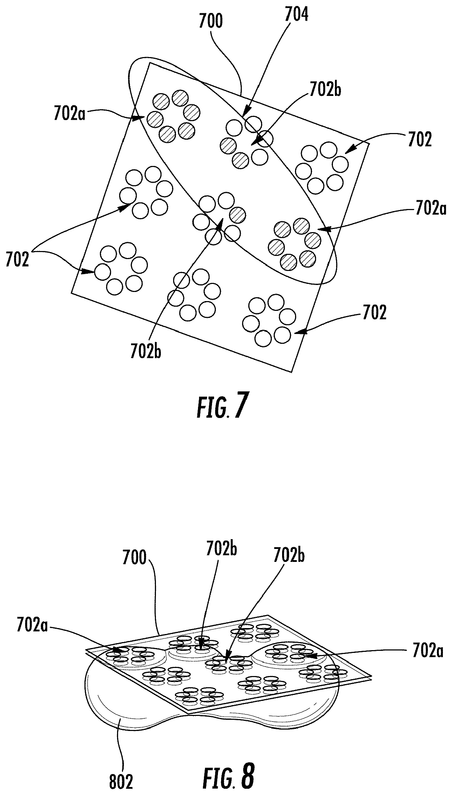

FIGS. 7-10 illustrate different examples of electrode array configurations in which electrodes are combined into one or more electrode groups, each acting as a larger electrode for ablation procedures. FIG. 7 illustrates a top view of an ablation contour of electrode array 700, in which individual electrode sets 702, used to provide high resolution mapping, are combined to simulate an ablation macro-electrode for ablation. One or more electrodes can be selected and combined (e.g., via a processor) with one or more electrodes to provide a larger ablation electrode.

The electrode array 700 shown in FIG. 7 includes a matrix of electrode sets 702 each including six individual electrodes. As shown in FIG. 7, different electrodes from the electrode sets 702 are grouped (e.g., selected via a processor) to form an ablation macro-electrode 704 represented by the ellipse shown in FIG. 7. As shown, the ablation macro-electrode 704 includes two electrode sets 702a having six electrodes, and two electrode sets 702b having four electrodes. Each of the electrodes in the macro-electrode 704 (i.e., in the ellipse) can be combined to behave as a single, large ablation electrode, or as a series of ablation electrodes.

FIG. 8 illustrates a side perspective view of the ablation contour of the electrodes array 700 shown in FIG. 7. FIG. 8 also shows an estimated lesion 802 resulting from ablation (e.g., at 60 degrees Celsius) of tissue using the electrode array 700.

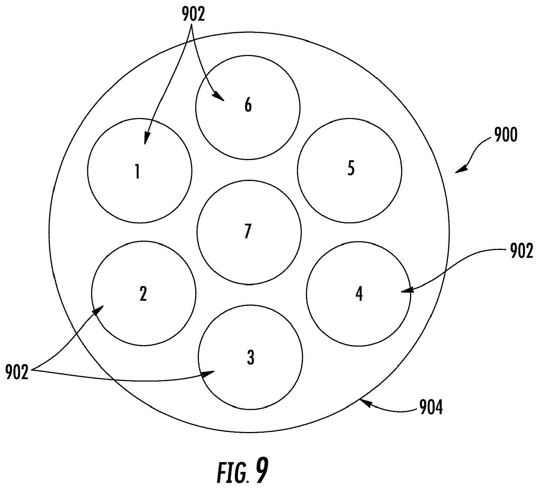

FIG. 9 is an illustration of another configuration of an electrode array 900, which includes seven electrodes 902 grouped in a circle to form a macro-electrode 904. The macro-electrode 904 simulates an ablation electrode in which the small electrodes 902 combine and obtain a combined power to be used during an ablation. The number of electrodes, and the circle shape of the electrode group shown in FIG. 9, is merely exemplary. Electrode arrays can include any number of electrodes grouped in configurations different from the configuration shown in FIG. 9 to provide a macro-electrode having power to be used during an ablation.

The electrode array configurations (e.g., number of electrodes, shapes of electrode groups) shown in FIGS. 7-9 are merely exemplary. Embodiments may include electrode arrays having any number of electrodes grouped in configurations different from the configurations shown in FIGS. 7-9 to provide a macro-electrode having power to be used during an ablation. During the ablation procedure, any number of electrodes, forming any shape, may be grouped (e.g., selected via a processor) to form a macro-electrode for ablation.

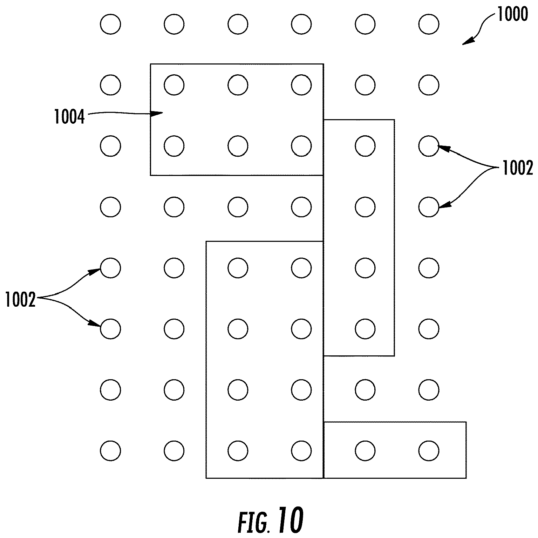

FIG. 10 is an illustration of another configuration of an electrode array 1000. As shown in FIG. 10, electrode array 1000 includes a matrix of electrodes 1002 equidistant from each other. As shown in FIG. 10, a portion of the electrodes 1002 are grouped together to form macro-electrode 1004 or as a group of macro electrodes used to form a contingent ablation curve.

Any of the functions and methods described herein can be implemented in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

## Claims

1. A medical apparatus used to acquire electrical activity of patient anatomy, the apparatus comprising:
an elongated body; and
a tip portion coupled to the elongated body, the tip portion comprising one or more inflatable sections, each inflatable section having a plurality of electrodes disposed on one of: (i) an outer surface of the one or more inflatable sections; and (ii) an inner surface and the outer surface of the one or more inflatable sections,
wherein the one or more inflatable sections, when inflated, cause a portion of the plurality of electrodes to contact a surface of an organ and provide a pathway for physiological fluid to flow through the tip portion.

2. The apparatus according to claim 1, wherein the tip portion is a tulip balloon portion and the one or more inflatable sections comprise one or more holes extending through the one or more inflatable sections and configured to provide the pathway for physiological fluid to flow through the tip portion.

3. The apparatus according to claim 1, wherein the organ is a heart and the one or more inflatable sections, when inflated, cause the plurality of electrodes to contact an inner surface of a heart chamber.

4. The apparatus according to claim 3, wherein the one or more inflatable sections comprise:
a first inflatable section; and
a second inflatable section comprising one or more protrusions extending from an inner surface of the second inflatable section,
wherein, in a first state, the first inflatable section is configured to contact the one or more protrusions and flex, causing the plurality of electrodes to contact the inner surface of the heart chamber and cause a portion of the outer surface of the first inflatable section to be spaced from the inner surface of the heart chamber and provide the pathway for the physiological fluid to flow.

5. The apparatus according to claim 4, wherein in a second state, the first inflatable section and the second inflatable section are spaced from each other and form a gap between each other to provide the pathway for the physiological fluid to flow.

6. The apparatus according to claim 1, wherein the one or more inflatable sections are concentrically wound inflatable sections and, when inflated, each concentrically wound inflatable section is configured to form a gap to provide the pathway for the physiological fluid to flow.

7. The apparatus according to claim 1, wherein the plurality of electrodes are configured to acquire electrical signals from the surface of the organ during a mapping procedure and during an ablation procedure.

8. The apparatus according to claim 1, wherein the one or more inflatable sections are configured to provide electrode coverage to acquire electrical activity of each of a plurality of portions of the organ while preventing physiological fluid restriction.

9. A medical apparatus used to acquire electrical activity of patient anatomy, the apparatus comprising:
an elongated body; and
a tulip balloon tip portion coupled to the elongated body, the tulip balloon tip portion comprising:
a plurality of inflatable sections configured to provide a pathway for physiological fluid to flow through the tip portion, the plurality of inflatable sections having a plurality of electrodes disposed on at least one of (i) an outer surface of the plurality of inflatable sections; and (ii) an inner surface and the outer surface of the plurality of inflatable sections,
the plurality of inflatable sections comprising:
a first inflatable section; and
a second inflatable section,
wherein the second inflatable section is configured to overlap the first inflatable section in a first state.

10. The apparatus according to claim 9, wherein the plurality of electrodes are configured to contact an inner surface of a heart and acquire electrical signals from the inner surface of a heart chamber during a mapping procedure and during an ablation procedure.

11. The apparatus according to claim 9, wherein the first inflatable section comprises a pair of opposing first inflatable sections, the second inflatable section comprises a pair of opposing second inflatable sections, and each one of the pair of opposing second inflatable sections are configured to overlap the pair of opposing first inflatable sections in the first state.

12. The apparatus according to claim 11, wherein each of the pair of opposing second inflatable sections comprise one or more protrusions extending from an inner surface of each of the pair of opposing second inflatable sections, and
in the first state, each of the pair of opposing first inflatable sections is configured to contact the one or more protrusions of adjacent opposing second inflatable sections and flex, causing a portion of the plurality of electrodes to contact an inner surface of a heart chamber and cause a portion of the outer surface of a corresponding inflatable section to be spaced from the inner surface of the heart chamber and provide the pathway for the physiological fluid to flow.

13. The apparatus according to claim 12, wherein the one or more protrusions comprise a pair of ridges extending along the inner surface between one end of the one of the opposing first inflatable section and a second end of the opposing first inflatable section.

14. The apparatus according to claim 9, wherein in a second state, the plurality of electrodes which are disposed on the outer surface contact an inner surface of a heart chamber and the first inflatable section is spaced from the second inflatable sections forming a gap to provide the pathway for the physiological fluid to flow.

15. The apparatus according to claim 9, wherein one or more of the plurality of inflatable sections comprise one or more holes to provide the pathway for the physiological fluid to flow.

16. A medical apparatus used to acquire electrical activity of patient anatomy, the apparatus comprising:
an elongated body; and
an inflatable tip portion coupled to the elongated body, the inflatable tip portion comprising:
one or more concentrically wound inflatable sections, each having a plurality of electrodes disposed on at least one of: (i) an outer surface of a corresponding inflatable section; and (ii) an inner surface and the outer surface of the corresponding inflatable section,
wherein, when inflated, the one or more concentrically wound inflatable sections are configured to form a gap to provide a pathway for physiological fluid to flow and the plurality of electrodes are caused to contact an inner surface of an organ.

17. The apparatus according to claim 1, claim 9 or claim 16, wherein the plurality of electrodes includes a group of electrodes which forms an ablation electrode.

18. The apparatus according to claim 16, wherein, when inflated, the plurality of electrodes disposed on the outer surface of each concentrically wound inflatable section are configured to acquire electrical signals from the surface of a heart chamber during a mapping procedure and during an ablation procedure.
